# EUROPÄISCHE PATENTSCHRIFT

(11) **EP 0 797 775 B1**
(45) Veröffentlichungstag und Bekanntmachung des Hinweises auf die Patenterteilung: **26.05.1999**
(21) Anmeldenummer: 95941688.4
(22) Anmeldetag: 07.12.1995
(51) Int. Cl.: G01N 33/543, G01N 33/52, G01N 33/569, C12Q 1/04

(54) **TESTSET ZUR ANALYSE VON KÖRPERFLÜSSIGKEITEN, VERFAHREN ZU SEINER HERSTELLUNG UND ANALYSEVERFAHREN**
TEST SET FOR ANALYSIS OF BODY FLUIDS, PROCESS FOR PRODUCING IT AND ANALYTICAL PROCEDURE
TROUSSE D'ESSAI POUR ANALYSE DE FLUIDES ORGANIQUES, PROCEDE POUR SA FABRICATION ET MODE OPERATOIRE D'ANALYSE

(30) Priorität: 16.12.1994 DE 4444764
(43) Veröffentlichungstag der Anmeldung: 01.10.1997
(73) Patentinhaber: M & M DENTAL-MEDIZIN GMBH, 47443 Moers (DE)
(72) Erfinder: MÖLL, Claus-Jürgen, D-47443 Moers (DE); PAESSENS, Theodor, D-47546 Kalkar (DE); ALTHOFF, Jörg, D-48268 Greven (DE)
(74) Vertreter: Schoenen, Norbert
(86) Internationale Anmeldenummer: EP9504812
(87) Internationale Veröffentlichungsnummer: WO9618902

(56) Entgegenhaltungen:
- EP-A- 0 418 739
- EP-A- 0 442 231
- DE-A- 3 632 303
- DE-A- 3 709 497

## Beschreibung

Die Erfindung betrifft ein Testset zur Analyse von Körperflüssigkeiten, insbesondere des Speichels.

Zur Kariesprophylaxe ist eine regelmäßige Vorsorgeuntersuchung beim Zahnarzt erforderlich. Empfohlen wird im allgemeinen ein halbjährlicher Rhythmus. Diese Empfehlung wird jedoch häufig nicht eingehalten, da der medizinische Laie sein persönliches Kariesrisiko nicht kennt und oft irrtümlich glaubt, bei ihm selber sei dieses Risiko gering.

Nützlich wäre daher ein Test, den der Laie selber durchführen kann und der ihm auf einfache, schnelle und preiswerte Weise Auskunft über sein persönliches Kariesrisiko gibt.

Die genauen Ursachen von Zahnkaries und die dabei ablaufenden Vorgänge sind noch nicht vollständig geklärt. Fest steht jedoch, daß die Zahnkaries an das Vorhandensein von Mikroorganismen und Kohlenhydraten in der Mundhöhle gebunden ist. Durch Vergärung der kohlenhydrathaltigen Speisereste, die an den Zähnen haften, kommt es zur Ansäuerung des Milieus, vor allem durch das Abbauprodukt Milchsäure, und damit zum Herauslösen anorganischer Salze aus dem Zahnhartgewebe. In das aufgelockerte Hartgewebe können Mikroorganismen einwandern, die das organische Stützgewebe des Zahns angreifen. Die Zahnkaries beginnt vor allem an Stellen mit Zahnbelag (Plaque).

Untersuchungen der Erfinder haben gezeigt, daß die Analyse des Speichels, insbesondere seines pH-Werts, Auskunft über Kariesrisiko geben kann, wie unten näher ausgeführt ist.

Speicheltests und Hilfsmittel zum Entnehmen von Speichel für Analysezwecke sind seit längerem bekannt. Ein derartiges Verfahren ist in der DE 36 32 303 Al beschrieben. Hier wird ein elastischer, saugfähiger, inerter Körper vom Probanden gekaut und anschließend in ein mit einem gelochten Boden versehenes Zentrifugenröhrchen eingebracht. Dieses Verfahren ist jedoch wie viele andere bekannte Verfahren nur für die ärztliche Praxis geeignet und kann nicht ohne weiteres vom Laien selber durchgeführt werden.

Aus dem Stand der Technik ist außerdem ein Mundtupfer für die Mundhygiene bekannt (DE 37 09 497 A1). An dem einem Ende des Stützstäbchens des Mundtupfers ist ein Tupfer angebracht, der mit einem Mundbehandlungsmittel getränkt ist. Ein Verdunsten des Mundbehandlungsmittels wird durch einen trockenen, speichellöslichen polymeren Überzug des Tupfers verhindert, so daß der Mundtupfer über längere Zeit gelagert werden kann, ohne seine Gebrauchsfähigkeit zu verlieren. Das Überzugsmaterial besteht im wesentlichen aus Acrylharz und enthält keine Kohlenhydrate. Anders als in der vorliegenden Erfindung dient der bekannte Mundtupfer zum Einbringen von Flüssigkeit in die Mundhöhle, aber nicht zur Entnahme von Speichel.

Die DE 29 41 471 A1 beschreibt einen Provokationstest auf Zahnkaries, arbeitet aber mit einem flüssigen Testmittel. Das Testmittel selber enthält nicht nur das Kohlenhydrat, sondern auch den pH-Indikator. Nachteilig ist insbesondere die umständliche Anwendung. Es ist nämlich notwendig, den vom Patienten entnommenen Zahnbelag in das Testmittel einzuarbeiten und die Farbveränderung des Mittels nach einer Inkubation über eine feste Zeitspanne, zum Beispiel 30 Minuten, zu beobachten.

Die US 5 357 989 beschreibt ein Zahnreinigungsmittel, zum Beispiel Zahnseide, das mit einem pH-Indikator imprägniert oder beschichtet ist. Es handelt sich hier nicht um einen Provokationstest, sondern nur um die Messung des aktuellen pH-Werts.

Eine weitere wäßrige Reagenz zum Durchführen eines Tests auf Zahnkaries ähnlich wie in der DE 29 41 471 A1 wird in der DE 30 48 705 A1 näher beschrieben. Es handelt sich hier ebenfalls um einen Provokationstest, da die wäßrige Reagenzlösung auch Saccharose enthalten kann. Nachteilig ist aber auch hier die relativ lange Wartezeit nach der Zugabe des Speichels in die Testlösung, die mit etwa 30 Minuten angegeben wird. Für Laien ist ein solches Verfahren unbequem und umständlich.

Die EP 0 097 904 A1 arbeitet mit einem Papier, das mit Sucrose und einem pH-Indikator imprägniert ist. Zur Durchführung des Testes wird Speichel auf dieses Papier aufgebracht. Auch hier ist eine Inkubation während einer Zeitdauer von 20 bis 30 Minuten notwendig, die einen großen Nachteil bei der Anwendung durch Laien darstellt.

Ein weiteres Testmittel wird in der WO 91/14000 beschrieben. Das Testmittel besteht aus einem Träger mit einem ersten porösen Bereich, nämlich einem Filterpapier, das mit einer Farbtest-Substanz imprägniert ist. Ein zweiter poröser Bereich ist mit einem Farbentwickler imprägniert. Zur Anwendung wird Speichel auf den ersten Bereich aufgebracht und dann der Träger gefaltet, so daß die beiden Bereiche miteinander in Kontakt kommen. Nachteilig ist auch hier die Notwendigkeit einer Inkubation bei einer Temperatur von etwa 37 °C während einer Zeitdauer von typischerweise 15 Minuten. Allein die Notwendigkeit einer bestimmten Mindesttemperatur macht diesen Test für den Laien in der Anwendung unbrauchbar.

Auch der Test nach der US 3 746 624 erfordert eine Inkubation der Probe, und zwar in diesem Fall in einer Lösung für etwa 48 Stunden.

Schließlich sei noch die US 4 976 951 genannt. Hier wird ein zuckerhaltiges Kaugummi vom Probanden einige Zeit gekaut, dann gewaschen und schließlich bei 35 bis 37 °C für 6 bis 24 Stunden in einer speziellen Flüssigkeit bebrütet. Ein derartiges Testmittel ist für den Laien in der Handhabung äußerst umständlich.

Der Erfindung liegt daher die Aufgabe zugrunde, ein preiswertes Testset zur Analyse von Körperflüssigkeiten, insbesondere von Speichel, zu entwickeln, das vom medizinischen Laien auf bequeme, angenehme, einfache, schnelle und sichere Weise zu handhaben ist und einen sogenannten Provokationstest ermöglicht.

Diese Aufgabe wird beim eingangs genannten Testset erfindungsgemäß gelöst durch einen Träger mit einem saugfähigen Material, insbesondere trockener medizinischer Watte, zur Entnahme der Körperflüssigkeit und mit einer von der Körperflüssigkeit löslichen, eine bestimmte Reaktion des Körpers und/oder seiner Mikroorganismen provozierenden Masse und durch eine Einrichtung zum Bestimmen medizinisch relevanter Eigenschaften der entnommenen Körperflüssigkeit, insbesondere des pH-Werts.

Das erfindungsgemäße Testset ist im Vergleich zum Stand der Technik sehr einfach, schnell und angenehm anwendbar. Der Proband führt dabei einen insbesondere zuckerhaltigen Träger in seinen Mund ein. Es ist nicht notwendig, daß ein chemischer Indikator oder sonstige, für Laien unangenehme Chemikalien in die Mundhöhle gelangen müssen. Wartezeiten müssen - bis auf das angenehme Lutschen der die Reaktion provozierenden Masse im Fall des Zahnkaries-Test - nicht eingehalten werden. Eine Inkubation einer entnommenen Probe ist nach der Entnahme der Körperflüssigkeit nicht notwendig. Die für die provozierte Reaktion erforderliche Wartezeit läuft nämlich im Gegensatz zum Stand der Technik bereits während der Auflösung der provozierenden Masse in der Körperflüssigkeit ab, also bereits vor der Entnahme der Flüssigkeit mit dem saugfähigen Material.

Nachdem der Träger mit der Körperflüssigkeit in Berührung gekommen ist, dauert es eine gewisse Zeit, bis die daran haftende Masse aufgelöst ist. In diesem Zeitraum wird der die Körperreaktion hervorrufende Bestandteil der Masse abgegeben, und der Körper reagiert darauf in charakteristischer Weise.

Dieser Zeitraum läßt sich durch eine geeignete Wahl der Menge und der Zusammensetzung der Masse vorgeben, so daß der Anwender nicht auf das Einhalten einer bestimmten Zeitdauer zu achten braucht. Es ist nur erforderlich, den Träger bis zur vollständigen Auflösung der Masse mit der Körperflüssigkeit in Kontakt zu halten.

Nachdem sich die Masse aufgelöst hat, wird die Körperflüssigkeit vom saugfähigen Material absorbiert und kann mit Hilfe des Trägers auf die Einrichtung zum Bestimmen medizinisch relevanter Eigenschaften aufgebracht werden, zum Beispiel durch Abrollen des feuchten Endes eines Stäbchens auf pH-Papier. Durch Vergleich der Verfärbung mit farbigen Vergleichsfeldern läßt sich der pH-Wert des Speichels sofort, schnell und sicher ablesen.

Vorzugsweise ist die Masse als Überzug für das saugfähige Material ausgebildet. Insbesondere umschließt der Überzug das saugfähige Material vollständig. Zum einen wird damit ein Schutz des in der Regel keimfreien saugfähigen Materials erreicht. Zum anderen nimmt das saugfähige Material die Körperflüssigkeit erst dann auf, wenn bereits ein Anteil des Überzugs oder sogar der gesamte Überzug sich in der Körperflüssigkeit aufgelöst hat und somit eine bestimmte Wartezeit verstrichen ist, in der der Körper und/oder seine Mikroorganismen auf die Inhaltsstoffe der Masse in der gewünschten Weise reagiert haben.

Um dem Anwender das Einhalten der für die Reaktion des Körpers und/oder seiner Mikroorganismen erforderlichen Wartezeit zu erleichtern, wird weiterhin vorgeschlagen, daß die Zusammensetzung und Menge der Masse derart gewählt worden ist, daß bei vollständiger Auflösung der Masse durch die Körperflüssigkeit die Mindestzeitdauer zum Auslösen der Reaktion des Körpers und/oder seiner Mikroorganismen vergangen ist. Wenn der Anwender daher feststellt, daß sich die Masse vollständig aufgelöst hat, kann der Träger entnommen werden, und die gewünschten medizinisch relevanten Eigenschaften der entnommenen Körperflüssigkeit können mittels der oben genannten Einrichtung bestimmt werden.

Die Erfindung findet bevorzugt Anwendung beim Bestimmen des pH-Werts von Speichel, wobei der Stoffwechsel der Mikroorganismen in der Mundhöhle zuvor durch die im Überzug enthaltenen Stoffe, insbesondere durch Kohlenhydrate angeregt worden ist. Der Stoffwechsel führt zu einem Absinken des pH-Werts, also zu einem stärker saurem Milieu. Die Messung des pH-Werts erlaubt damit ein Rückschluß auf die Anzahl der für die Kariesbildung verantwortlichen Mikroorganismen in der Mundhöhle. Damit läßt sich nach den Untersuchungen der Erfinder das Kariesrisiko relativ genau bestimmen. Die gemessenen pH-Werte korrelieren stark mit den ermittelten Plaque-Indices, die wiederum in engem Zusammenhang mit dem Kariesrisiko stehen.

Die Erfindung ist jedoch nicht auf die Verwendung des Testsets zum Bestimmen des pH-Werts von Speichel beschränkt. Das Testset kann vielmehr für alle Provokationstests eingesetzt werden, also für Testverfahren, bei denen der menschliche Körper einschließlich seiner Mikroorganismen durch Zugabe bestimmter Stoffe zunächst zu einer Reaktion provoziert wird. Die infolge der Reaktion veränderte Körperflüssigkeit wird dann analysiert.

In einer besonders vorteilhaften Ausgestaltung der Erfindung zum Bestimmen des Kariesrisikos enthält die Masse mindestens einen den Stoffwechsel der Mikroorganismen in der Mundhöhle anregenden Inhaltsstoff, insbesondere einen zuckerartigen Stoff oder andere Kohlenhydrate, und gegebenenfalls zusätzlich einen Zuckerersatzstoff. Als Beispiel für den zuckerartigen Stoff seien Saccharose und Glucose genannt.

Bei einem Testset zum Bestimmen des Kariesrisikos wird weiterhin vorgeschlagen, daß die Zusammensetzung und Menge der Masse derart gewählt worden ist, daß sie sich in der Mundhöhle während eines Zeitraums von 1 bis 10 min und vorzugsweise von 2 bis 5 min auflöst. Nach der Auflösung der Masse, die insbesondere als Überzug für das saugfähige Material ausgebildet ist, ist der Anwender sicher, daß die zum Stimmulieren eines verstärkten Stoffwechsels der für den Zahnkaries verantwortlichen Mikroorganismen erforderliche Wartezeit vergangen ist.

Weiterhin wird vorgeschlagen, daß die Masse aus einer Glasur besteht, die sich im wesentlichen aus dem den Stoffwechsel der Mikroorganismen anregenden Inhaltsstoff und Fett als Bindemittel zusammensetzt. Ein derartiger Überzug ist in der Massenproduktion des Testsets auf besonders wirtschaftliche Weise auf das saugfähige Material aufzubringen.

Insbesondere wird bei einem Testset zum Bestimmen des Kariesrisikos vorgeschlagen, daß die Masse neben Aromastoffen aus
17 bis 45 Gew.-% Glucose
8 bis 45 Gew.-% Gelatine
0 bis 70 Gew.-% Konservierungsmittel
2 bis 10 Gew.-% Saccharose
besteht.

Bei dieser Zusammensetzung ist die Masse vorzugsweise als Überzug des saugfähigen Materials mit einer Dicke von 0,1 bis 0,5 mm, vorzugsweise von 0,1 bis 0,2 mm ausgebildet. In dieser Ausgestaltung des Testsets ist gewährleistet, daßnach der vollständigen Auflösung des Überzugs eine Wartezeit von 2 bis 5 min eingehalten worden ist.

Die Akzeptanz des Stäbchens beim Verbraucher wird verbessert, wenn die Masse Aromastoffe enthält. Als Beispiele sind die Aromen von Zitrone, Limone, Pfefferminze und Eukalyptus zu nennen.

Im Hinblick auf die Entsorgung ist es aus ökologischen Gründen günstig, wenn der Träger als Stäbchen ausgebildet ist, das im wesentlichen aus Holz besteht.

Wenn das saugfähige Material aus Einzel fasern, insbesondere aus medizinischer Watte, besteht, wird ein für den Anwender unangenehmes Zurückbleiben von einzelnen Fasern des saugfähigen Materials im Mund auf sichere Weise verhindert, wenn das Material von einem Textil umgeben ist, das mit der speichellöslichen Masse beschichtet ist. Aus Ökologischen Gründen ist das Textil als ein Tuch aus Naturfasern, vorzugsweise aus Baumwolle, ausgebildet.

Die Akzeptanz des Testsets insbesondere bei Kindern läßt sich steigern, wenn das saugfähige Material die Form eines Tieres, Tierkopfes, Kinderspielzeugs oder eines anderen für Kinder ansprechenden Gegenstandes hat.

In einer besonders preiswerten und für den Verbraucher leicht zu handhabenden Ausführung ist die Einrichtung zum Bestimmen des pH-Werts des entnommenen Speichels als Testpapier zum Bestimmen des pH-Werts ausgebildet. So kann eine Testzone vorgesehen sein, die sich beim Aufbringen von Flüssigkeiten mit unterschiedlichem pH-Wert unterschiedlich verfärbt. Farbige Vergleichsfelder können eine schnelle und sichere Bestimmung des pH-Werts ermöglichen. Derartige Testpapiere sind infolge der Herstellung in hoher Stückzahl sehr preiswert.

Die Erfindung betrifft außerdem ein Verfahren zum Analysieren des Speichels mit dem beschriebenen Testset. Erfindungsgemäß wird vorgeschlagen, daß man das saugfähige Material sowie die Masse solange im Mund behält, bis sich der Überzug aufgelöst hat und dann mit der Einrichtung den pH-Wert des entnommenen Speichels bestimmt. Daraus lassen sich dann Rückschlüsse auf die Mikroorganismen in der Mundhöhle und damit auf das Kariesrisiko schließen, wie,bereits erläutert worden ist.

Die Erfindung betrifft schließlich noch ein wirtschaftliches Verfahren zum Herstellen des erfindungsgemäßen Testsets. Vorzugsweise wickelt man zunächst das angefeuchtete saugfähige Material, insbesondere medizinische Watte, um ein Ende eines Stäbchens, preßt es gegebenenfalls in eine bestimmte Form, taucht dieses Ende nach einer Trocknung des Stäbchens in eine verflüssigte, insbesondere geschmolzene Überzugsmasse und läßt den Überzug schließlich erhärten.

Im folgenden wird die Erfindung anhand von Zeichnungen und eines Ausführungsbeispiels näher beschrieben. Es zeigen
- Figur 1: einen Querschnitt eines Stäbchens eines erfindungsgemäßen Testsets,
- Figur 2: ein Diagramm des pH-Werts über der Tageszeit in Abhängigkeit von der Mundhygiene und
- Figur 3: die Abhängigkeit des Plaque-Index von der Mundhygiene.

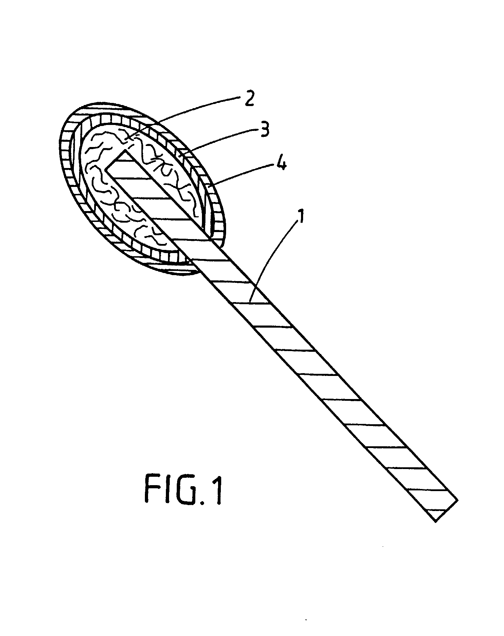

Ein Ende eines in Figur 1 gezeichneten, aus Holz bestehenden Stäbchens 1 ist mit medizinischer Watte 2 umwickelt. Ein Baumwolltuch 3 umhüllt diese Fasermasse und verhindert damit das Ablösen einzelner Fasern bei Kontakt der Fasermasse mit dem Speichel. Das Tuch 3 ist mit einer Glasur 4 beschichtet, die die Substanz zur Anregung des Stoffwechsels der Mikroorganismen sowie Fett als Bindemittel enthält.

Die Glasur hat die folgende Zusammensetzung:
48 Gew.-% Glucose
40 Gew.-% Gelatine
3 Gew.-% Natriumbenzoat als Konservierungsmittel
9 Gew.-% Saccharose sowie Aromastoffe (Zitrone, Waldhimbeer).

Die Dicke des Überzugs beträgt 0,1 bis 0,2 mm. Sie ist so gewählt, daß sich der Überzug in der Mundhöhle nach etwa 3 min aufgelöst hat.

Beim Herstellen des Stäbchens wird zunächst das eine Ende eines Holzstäbchens mit feuchter Watte umwickelt, so daß sie fest am Holz haftet. Dieser Schritt ist aus der Herstellung von Wattestäbchen bekannt, die zum Reinigen von Ohren verwendet werden. Nach dem Trocknen wird das Baumwolltuch aufgebracht, und das Ende des Holzstäbchens wird in eine durch Erwärmen verflüssigte Glasur der genannten Zusammensetzung getaucht.

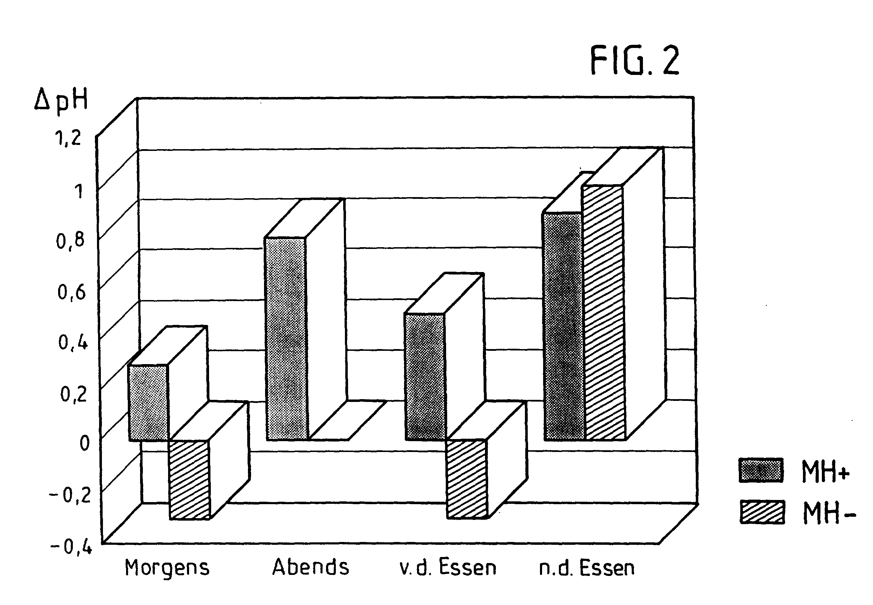

Figur 2 zeigt die Abweichung des pH-Werts des Speichels vom neutralen Wert pH = 7 in Abhängigkeit von der Mundhygiene, abgekürzt durch MH. Die linken Balken jeder Gruppe zeigen den Wert mit, die rechten Balken ohne Mundhygiene. Untersuchungen von 100 Probanden ergaben abends eine durchschnittliche Abweichung des pH-Werts von + 0,8 auf 7,8, morgens jedoch von nur + 0,3 auf 7,3. Die Standardabweichung betrug ± 15 %. Die Veränderung des pH-Werts ist auf das Wachstum der Mikroorganismen und deren Stoffwechsel in der Nacht zurückzuführen. Das gilt auch für Probanden mit einer durchschnittlichen Mundhygiene.

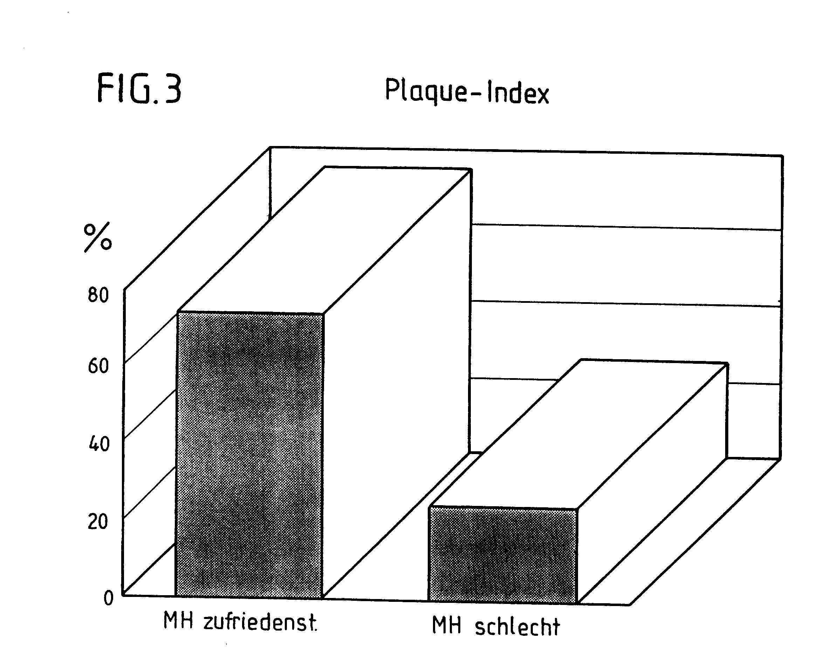

Morgens und abends findet eine deutliche Übersäuerung statt. Nach dem Reinigen der Zähne und der Reaktion der Speicheldrüsen tritt eine rasche Alkalisierung auf, die nach der Aufnahme von Speisen wieder schnell verschwindet. Die Übersäuerung infolge fehlender oder mangelhafter Mundhygiene korreliert in auffälliger Weise mit den ermittelten Plaque-Indices, wie das Diagramm in Figur 3 zeigt. Der in Prozent angegebene Plaque-Index ist ein Maß für das Vorhandensein von Zahnbelag. Je stärker der Zahnbelag ist, desto niedriger ist der Plaque-Index.

Wird nun im erfindungsgemäßen Test der Stoffwechsel der Mikroorganismen durch die Zuckerstoffe im Überzug des Stäbchens provoziert, sinkt der pH-Wert des Speichels um so stärker ab, je größer die Anzahl der schädlichen, Karies verursachenden Mikroorganismen ist. Die Messung des pH-Werts nach der Zugabe von Zuckerstoffen oder anderen Kohlenhydraten gibt daher Aufschluß über das Kariesrisiko, erlaubt eine Erfolgskontrolle der Mundhygiene und zeigt außerdem an, obeine, eventuell zusätzliche Zahnreinigung notwendig ist.

Zur Anwendung des Testset wird das oben genannte Stäbchen aus einem Reagenzglas entnommen und in den Mund eingeführt. Nach dem vollständigen Ablutschen des wohlschmeckenden Überzugs innerhalb von etwa 3 min wird das Stäbchen mit der mit Speichel getränkten Watte aus dem Mund entnommen. Die feuchte Watte wird nun auf einem Teststreifen kurz hin- und hergerollt, bis sich der Streifen verfärbt. Durch Vergleich der Verfärbung mit einer neben dem Teststreifen angeordneten Farbskala läßt sich der pH-Wert und damit das Kariesrisiko ablesen.

Vorzugsweise sind der Teststreifen und die Farbskala unmittelbar auf oder innerhalb der Verpackung des Stäbchens an einer gut zugänglichen Stelle angebracht, z. B. auf der Innenseite eines zusammengeklappten Papier- oder Papp-Heftchens.

## Patentansprüche

1. Testset zur Analyse von Körperflüssigkeiten, insbesondere des Speichels,
**gekennzeichnet durch**
einen Träger (1) mit einem saugfähigen Material (2), insbesondere trockener medizinischer Watte, zur Entnahme der Körperflüssigkeit und mit einer von der Körperflüssigkeit löslichen, eine bestimmte Reaktion des Körpers und/oder seiner Mikroorganismen provozierenden Masse (4), und durch eine Einrichtung zum Bestimmen medizinisch relevanter Eigenschaften der entnommenen Körperflüssigkeit, insbesondere des pH-Werts.

2. Testset nach Anspruch 1,
**dadurch gekennzeichnet**,
daß die Masse (4) als Überzug für das saugfähige Material (2) ausgebildet ist.

3. Testset nach dem vorhergehenden Anspruch,
**dadurch gekennzeichnet**,
daß der Überzug (4) das saugfähige Material (2) vollständig umschließt.

4. Testset nach einem der vorhergehenden Ansprüche,
**dadurch gekennzeichnet**,
daß die Zusammensetzung und Menge der Masse (4) derart gewählt worden ist, daß bei vollständiger Auflösung der Masse durch die Körperflüssigkeit die Mindestzeitdauer zum Auslösen der Reaktion des Körpers und/oder seiner Mikroorganismen vergangen ist.

5. Testset nach einem der vorhergehenden Ansprüche,
**dadurch gekennzeichnet**,
daß die Masse (4) mindestens einen den Stoffwechsel der Mikroorganismen in der Mundhöhle anregenden Inhaltsstoff, insbesondere einen zuckerartigen Stoff oder andere Kohlenhydrate, und gegebenenfalls zusätzlich einen Zuckerersatzstoff enthält.

6. Testset nach dem vorhergehenden Anspruch
**dadurch gekennzeichnet**,
daß die Zusammensetzung und Menge der Masse (4) derart gewählt worden ist, daß sie sich in der Mundhöhle während eines Zeitraums von 1 bis 10 min, vorzugsweise von 2 bis 5 min auflöst.

7. Testset nach einem der vorhergehenden Ansprüche,
**dadurch gekennzeichnet**,
daß die Masse (4) aus einer Glasur besteht, die sich im wesentlichen aus dem den Stoffwechsel der Mikroorganismen anregenden Inhaltsstoff und Fett als Bindemittel zusammensetzt.

8. Testset nach Anspruch 6,
**dadurch gekennzeichnet**,
daß die Masse neben Aromastoffen aus
17 bis 45 Gew.-% Glucose
8 bis 45 Gew.-% Gelatine
0 bis 70 Gew.-% Konservierungsmittel
2 bis 10 Gew.-% Saccharose
besteht.

9. Testset nach Anspruch 8,
**dadurch gekennzeichnet**,
daß die Masse als Überzug für das saugfähige Material mit einer Dicke von 0,1 bis 0,5 mm, vorzugsweise von 0,1 bis 0,2 mm ausgebildet ist.

10. Testset nach einem der vorhergehenden Ansprüche,
**dadurch gekennzeichnet**,
daß die Masse (4) Aromastoffe enthält.

11. Testset nach einem der vorhergehenden Ansprüche,
**dadurch gekennzeichnet**,
daß der Träger als Stäbchen (1) ausgebildet ist, das vorzugsweise im wesentlichen aus Holz besteht.

12. Testset nach einem der vorhergehenden Ansprüche,
**dadurch gekennzeichnet**,
daß das saugfähige Material (2) aus Einzelfasern, insbesondere aus medizinischer Watte, besteht und von einem Textil (3), insbesondere einem Tuch aus Naturfasern, vorzugsweise aus Baumwolle, umgeben ist, das mit der speichellöslichen Masse (4) beschichtet ist.

13. Testset nach einem der vorhergehenden Ansprüche,
**dadurch gekennzeichnet**,
daß das saugfähige Material (2) die Form eines Tieres, Tierkopfes, Kinderspielzeugs oder eines anderen für Kinder ansprechenden Gegenstandes hat.

14. Testset nach einem der vorhergehenden Ansprüche,
**dadurch gekennzeichnet**,
daß die Einrichtung zum Bestimmen des pH-Werts des entnommenen Speichels als Testpapier zum Bestimmen des pH-Werts ausgebildet ist, das insbesondere zusammen mit einer Farbskala auf oder innerhalb der Verpackung des genannten Trägers angebracht ist.

15. Verfahren zum Analysieren des Speichels mit dem Testset nach einem der vorhergehenden Ansprüche,
**dadurch gekennzeichnet**,
daß man das saugfähige Material (2) sowie die Masse (4) solange im Mund behält, bis sich die Masse (4) aufgelöst hat und dann mit der Einrichtung den pH-Wert des entnommenen Speichels bestimmt.

16. Verfahren zum Herstellen des Testsests nach einem der Ansprüche 1 bis 14,
**dadurch gekennzeichnet**,
daß man zunächst das angefeuchtete saugfähige Material (2), insbesondere medizinische Watte, um ein Ende eines Stäbchens (1) wickelt, gegebenenfalls in eine bestimmte Form preßt, nach einer Trocknung dieses Ende des Stäbchens in eine verflüssigte, insbesondere geschmolzene Überzugsmasse taucht und den Überzug (4) schließlich erhärten läßt.

## Claims

1. A test kit for analysing body fluids, more particularly saliva, characterized by a support (1) with an absorbent material (2), more particularly dry surgical cottonwool, for removing the body fluid and a mass (4) soluble in the body fluid and provoking a certain reaction of the body and/or its microorganisms and by a means for determining medically relevant properties of the body fluid removed, more particularly its pH value.

2. A test kit as claimed in claim 1, characterized in that the mass (4) is in the form of a coating for the absorbent material (2).

3. A test kit as claimed in the preceding claim, characterized in that the coating (4) completely surrounds the absorbent material (2).

4. A test kit as claimed in any of the preceding claims, characterized in that the composition and the quantity of the mass are selected so that, by the time the mass has been completely dissolved by the body fluid, the minimum time required to provoke the reaction of the body and/or its microorganisms has elapsed.

5. A test kit as claimed in any of the preceding claims, characterized in that the mass (4) contains at least one ingredient which stimulates the metabolism of the microorganisms in the mouth, more particularly a sugar-like substance or other carbohydrates, and optionally a sugar substitute.

6. A test kit as claimed in the preceding claim, characterized in that the composition and quantity of the mass (4) are selected so that the mass (4) dissolves in the mouth in 1 to 10 minutes and preferably in 2 to 5 minutes.

7. A test kit as claimed in any of the preceding claims, characterized in that the mass (4) consists of a glaze which is essentially composed of the ingredient which stimulates the metabolism of the microorganisms and a fat as the binder.

8. A test kit as claimed in claim 6, characterized in that the mass consists of flavourings and
17 to 45% by weight of glucose,
8 to 45% by weight of gelatine,
0 to 70% by weight of preservative,
2 to 10% by weight of sucrose.

9. A test kit as claimed in claim 8, characterized in that the mass is in the form of a coating for the absorbent material with a thickness of 0.1 to 0.5 mm and preferably 0.1 to 0.2 mm.

10. A test kit as claimed in any of the preceding claims, characterized in that the mass (4) contains flavourings.

11. A test kit as claimed in any of the preceding claims, characterized in that the support is in the form of a stick (1) which preferably consists essentially of wood.

12. A test kit as claimed in any of the preceding claims, characterized in that the absorbent material (2) consists of individual fibres, more particularly of surgical cottonwool, and is surrounded by a textile (3), more particularly a cloth of natural fibres, preferably cotton, which is coated with the salivasoluble mass (4).

13. A test kit as claimed in any of the preceding claims, characterized in that the absorbent material (2) is in the shape of an animal, an animal's head, a child's plaything or any other article attractive to children.

14. A test kit as claimed in any of the preceding claims, characterized in that the means for determining the pH value of the saliva removed is in the form of test paper for determining the pH value which is arranged on or in the kit pack, more particularly together with a colour scale.

15. A process for analysing saliva using the test kit claimed in any of the preceding claims, characterized in that the absorbent material (2) and the mass (4) are kept in the mouth until the mass (4) has dissolved, after which the pH value of the saliva removed is determined with the relevant means.

16. A process for the production of the test kit claimed in any of claims 1 to 14, characterized in that the moistened absorbent material (2), more particularly surgical cottonwool, is first wound around one end of a stick (1), optionally compressed into a certain shape and, after drying of the stick, the end in question is dipped into a liquefied, more particularly molten, coating composition and, finally, the coating (4) is left to harden.

## Revendications

1. Trousse d'essai pour l'analyse des liquides corporels, en particulier de la salive,
caractérisée par
un support (1) avec un matériau absorbant (2), avec en particulier de la ouate médicinale sèche, pour le prélèvement du liquide corporel et avec une masse soluble dans le liquide corporel, provoquant une réaction déterminée du corps et/ou de ses microorganismes (4), et
par un dispositif pour déterminer les propriétés médicalement pertinentes du liquide corporel prélevé, en particulier le pH.

2. Trousse d'essai selon la revendication 1,
caractérisée en ce que
la masse (4) est formée en revêtement pour le matériau absorbant (2) .

3. Trousse d'essai selon la revendication précédente,
caractérisée en ce que
le revêtement (4) entoure entièrement le revêtement absorbant (2).

4. Trousse d'essai selon l'une des revendications précédentes,
caractérisée en ce que
la composition et la quantité de la masse (4) sont choisies de manière que lorsque la masse se dissout entièrement dans le liquide corporel, il se soit écoulé la durée minimum pour le déclenchement de la réaction du corps et/ou de ses microorganismes.

5. Trousse d'essai selon l'une des revendications précédentes,
caractérisée en ce que
la masse (4) contient au moins un constituant renforçant le métabolisme des microorganismes dans la cavité buccale, en particulier un matériau saccharique ou d'autres hydrates de carbone, et le cas échéant en outre un succédané de sucre.

6. Trousse d'essai selon la revendication précédente,
caractérisée en ce que
la composition et la quantité de la masse (4) sont choisies de manière à ce qu'elle se dissolvent dans la cavité buccale dans un intervalle de 1 à 10 minutes, de préférence de 2 à 5 minutes.

7. Trousse d'essai selon l'une des revendications précédentes,
caractérisée en ce que
la masse se compose d'un émail qui se compose essentiellement du constituant stimulant le métabolisme des microorganismes et d'une matière grasse comme liant.

8. Trousse d'essai selon la revendication 6,
caractérisée en ce que
la masse se compose, outre les arômes,
17 à 45 % en poids du glucose
8 à 45 % en poids de gélatine
0 à 70 % en poids d'agent de conservation
2 à 10 % en poids de saccharose.

9. Trousse d'essai selon la revendication 8,
caractérisée en ce que
la masse est formée en revêtement pour le matériau absorbant, avec une épaisseur de 0,1 à 0,5 mm, de préférence de 0,1 à 0,2 mm.

10. Trousse d'essai selon l'une des revendications précédentes,
caractérisée en ce que
la masse (4) contient des arômes.

11. Trousse d'essai selon l'une des revendications précédentes,
caractérisée en ce que
le support est formé en bâtonnet (1) qui se compose de préférence essentiellement de bois.

12. Trousse d'essai selon l'une des revendications précédentes,
caractérisée en ce que
le matériau absorbant (2) est constitué de fibres isolées, en particulier d'ouate médicinale, et est entouré d'un textile (3), en particulier d'un voile de fibres naturelles, de préférence de coton qui est recouvert d'une masse (4) soluble dans la salive.

13. Trousse d'essai selon l'une des revendications précédentes,
caractérisée en ce que
le matériau absorbant a la forme d'un animal, d'une tête d'animal, d'un jouet d'enfant ou d'un autre objet suscitant l'intérêt des enfants.

14. Trousse d'essai selon l'une des revendications précédentes,
caractérisée en ce que
le dispositif de détermination du pH de la salive prélevé est formé en papier expérimental pour la détermination du pH qui est déposé en particulier avec une échelle de couleur sur ou à l'intérieur du conditionnement mentionné.

15. Procédé d'analyse de la salive avec la trousse d'essai selon l'une des revendications précédentes,
caractérisé en ce qu'
on garde en bouche le matériau absorbant (2) ainsi que la masse (4) suffisamment longtemps pour que la masse (4) se soit dissoute, puis qu'on détermine avec le dispositif le pH de la salive prélevé.

16. Procédé de fabrication de la trousse d'essai selon l'une des revendications 1 à 14,
caractérisé en ce qu'
on enroule tout d'abord le matériau absorbant humidifié (2), en particulier la ouate médicale, autour d'une extrémité d'un bâtonnet (1), le cas échéant en ce qu'on le presse en une forme déterminée, en ce qu'après séchage on plonge cette extrémité du bâtonnet dans une masse de revêtement liquéfiée, en particulier fondue et en ce qu'enfin on laisse durcir le revêtement (4).
